# EUROPEAN PATENT APPLICATION

(11) **EP 2 778 232 A1**
(43) Date of publication of application: **17.09.2014**
(21) Application number: 13001224.8
(22) Date of filing: 12.03.2013
(51) Int. Cl.: C12Q 1/02

(54) **Method for the detection of multiresistant gram-negative bacteria**

(71) Applicant: Universität Heidelberg, 69117 Heidelberg (DE)
(72) Inventor: Grundt, Alexander, Dr. rer. nat., 67134 Birkenheide (DE); Neumaier, Michael, Prof. Dr. med., 68535 Edingen-Neckarhausen (DE)
(74) Representative: Müller-Boré & Partner Patentanwälte PartG mbB

(57) **Abstract**

The present invention relates to a method for the detection of multiresistant gram-negative bacteria. The method of the present invention is based on determining temperature-dependent penicillin- or aminopenicillin-specific activity of beta-lactamases in the bacteria to be analyzed.

## Description

The present invention relates to a method for the detection of multiresistant gram-negative bacteria. The method of the present invention is based on determining temperature-dependent penicillin- or aminopenicillin-specific activity of beta-lactamases in the bacteria to be analyzed.

Multiresistant microorganisms, *i.e.* microorganisms that are resistant against a multitude of different antibiotics, pose a more and more serious problem in fields such as hygiene, human medicine, veterinary medicine, the production and processing of foodstuffs, and the analysis or processing of drinking water or soil. However, fast and cost-efficient methods for the direct detection of multiresistant microorganisms are so far scarce and not available at all for a broad range of microorganisms.

In human medicine, the detection of multiresistant microorganisms is of particular interest, especially in the treatment of sepsis which is a potentially fatal systemic infection with microorganisms, in most cases bacteria, that leads to a systemic inflammatory response. Immediate empirically based broad-spectrum antibiotic therapy is a cornerstone in treatment of sepsis. However, the early de-escalation of broad-spectrum antibiotic therapy based on knowledge of microbial identification and susceptibility not only reduces therapeutic costs, but also reduces the spread of antimicrobial resistance. Unfortunately, the turnaround time of classical antibiotic susceptibility testing (AST) based on microbial growth inhibition by single antibiotics is 7.5 to 14 h for direct AST from blood culture (BC) fluid or 31.5 to 36 h for indirect AST from subcultured bacteria. Moreover, these methods are cost- and labor-intensive.

Thus, the detection of multiresistant bacteria by conventional methods is a time-consuming process. Since the mortality in sepsis increases by 7% each hour in which the sepsis is not adequately treated, the test results often can not be generated early enough for the patient.

Accordingly, the technical problem underlying the present invention is to provide an accelerated method for the detection of multiresistant bacteria which could reduce sepsis mortality, reduce the use of broad-spectrum antibiotics in cases where no multiresistant bacteria are detected, and in turn reduce the spreading of resistances and nosocomial infections.

The solution to the above technical problem is achieved by the embodiments characterized in the claims.

In particular, the present invention relates to a method for the detection of multiresistant gram-negative bacteria, comprising the steps of:
(a) providing a sample containing gram-negative bacteria;
(b) determining the penicillin- or aminopenicillin-specific activity of beta-lactamases in said bacteria at at least two different temperatures; and
(c) determining whether the bacteria are multiresistant;
wherein the bacteria are multiresistant when the penicillin- or aminopenicillin-specific activity of beta-lactamases determined in step (b) is inversely proportional to the temperature at which said activity has been determined.

The term "multiresistant" as used herein relates to the fact that with the method of the present invention, gram-negative bacteria can be detected that are resistant to distinct drugs or chemicals of a wide variety of structure and/or function targeted at eradicating the bacteria.

The term "beta-lactamases" as used herein relates to enzymes that are produced by some bacteria and are responsible for their resistance to beta-lactam antibiotics like penicillins, cephalosporins or carbapenems. Respective antibiotics have a four-atom ring known as beta-lactam as a common element in their structure. Beta-lactamases can break this ring open and thus deactivate the molecule's antibacterial properties. Accordingly, the above term includes all bacterial enzymes that have the ability to open a beta-lactam ring. Further, the above term includes all bacterial enzymes that can inactivate ordinary beta-lactam antibiotics (such as e.g. penicillins and aminopenicillins) and/or complex beta-lactam antibiotics (such as e.g. cephalosporins and piperacillin) and/or broad spectrum beta-lactam antibiotics (such as e.g. third generation cephalosporins) and/or carbapenems or other related so-called drugs of last resort. Many beta-lactamases are known in the art, including penicillinases, aminopenicillinases, cephalosporinases, broad-spectrum beta-lactamases, extended spectrum beta-lactamases (ESBL), carbapenemases, carbenicillinases, cloxacilanases, and metallo-beta-lactamases.

The term "penicillin- or aminopenicillin-specific activity of beta-lactamases" as used herein refers to the activity of beta-lactamases in opening the beta-lactam ring of penicillins or aminopenicillins.

In preferred embodiments, the gram-negative bacteria to be analyzed by the method of the present invention are known to express at least one beta-lactamase, and/or are known to be resistant to ampicillin, a beta-lactam antibiotic that is part of the aminopenicillin family.

The penicillin- or aminopenicillin-specific activity of beta-lactamases is determined in step (b) of the method of the present invention at at least two different temperatures, preferably at at least three different temperatures, and more preferably at at least four different temperatures. In a particular example, said activity is determined at ten different temperatures. Suitable temperatures are preferably chosen within the range of from 15°C to 43°C.

Methods for determining the penicillin- or aminopenicillin-specific activity of beta-lactamases in gram-negative bacteria are not particularly limited and are known in the art. Preferably, the penicillin- or aminopenicillin-specific activity of beta-lactamases is determined via the speed of ampicillin hydrolysis by the bacteria to be analyzed. A particular example of a method for determining penicillin- or aminopenicillin-specific activity of beta-lactamases via the speed of ampicillin hydrolysis is a recently described mass spectrometry-based assay for antibiotic susceptibility testing (MAAST). Briefly, in MAAST, the bacteria to be analyzed are incubated together with ampicillin. Over the course of e.g. one hour, samples are taken and quantitatively analyzed for ampicillin and specific degradation products thereof. The rate of decrease of ampicillin and/or the rate of increase of the degradation products is then used as a measure for penicillin-or aminopenicillin-specific activity of beta-lactamases. Preferably, a ratio between the rate of decrease of ampicillin and the rate of increase of the degradation products, or vice versa, is then used as a measure for penicillin- or aminopenicillin-specific activity of beta-lactamases.

In step (c) of the method of the present invention, it is determined whether the analyzed bacteria are multiresistant or not. In particular, when the penicillin- or aminopenicillin-specific activity of beta-lactamases determined in step (b) is inversely proportional to the temperature at which said activity has been determined, *i.e.* when the penicillin- or aminopenicillin-specific activity of beta-lactamases at the lower temperature or temperatures is higher then at the higher temperature or temperatures, this indicates multiresistance of said bacteria.

The gram-negative bacteria provided in step (a) of the present invention can be contained in any kind of sample, e.g. in swab samples such as wound swabs, in primary cultures such as blood cultures, or in subcultures derived from patients, food samples, water samples, soil samples or the like.

The present invention has been realized based on the finding that multiresistance of gram-negative bacteria correlates with the fact that penicillin-or aminopenicillin-specific activity of beta-lactamases of said bacteria is inversely proportional to the temperature at which said activity has been determined. In particular, the present invention is based on the finding that the active center of beta-lactamases that are exclusively specific for ampicillin is highly specific and hydrolyses ampicillin in a highly efficient manner in a wide range of temperatures. In contrast, broad spectrum beta-lactamases bind, besides ampicillin, also considerably larger molecules such as cephalosporins or piperacillin. This leads to a decreased specificity for ampicillin and a reduced rate of hydrolysis. With increasing temperatures, the entropy and motion of the molecules increases, leading to a faster abolishment of low specificity binding. Thus, the affinity of ampicillin to the active center of broad spectrum beta-lactamases decreases with increasing temperatures and, as a consequence, also the rate of hydrolysis decreases. The method of the present invention is able to deliver results in less than one hour and is further very inexpensive. Thus, it is suited for comprehensive screening in a wide range of potential applications. Compared to clinical standard processes for the detection of multiresistant bacteria, it provides up to 95% better economy of time.

The figure shows:

### Figure 1:

Temperature-dependent hydrolysis of ampicillin by six *E. coli* strains (samples 91, 92, 93, 111, 112, and 113) and one negative control (sample 0). Shown are the AUC (area under the curve) of ampicillin, which directly correlates to the ampicillin concentration (y-axis), and the temperature in °C (x-axis). Multi-resistant *E. coli* show a direct correlation between residual ampicillin and temperature between 27°C and 43°C caused by an inverse correlation between ampicillin-specific activity of beta-lactamases and temperature.

The present invention will be further illustrated in the following example without being limited thereto.

### Example

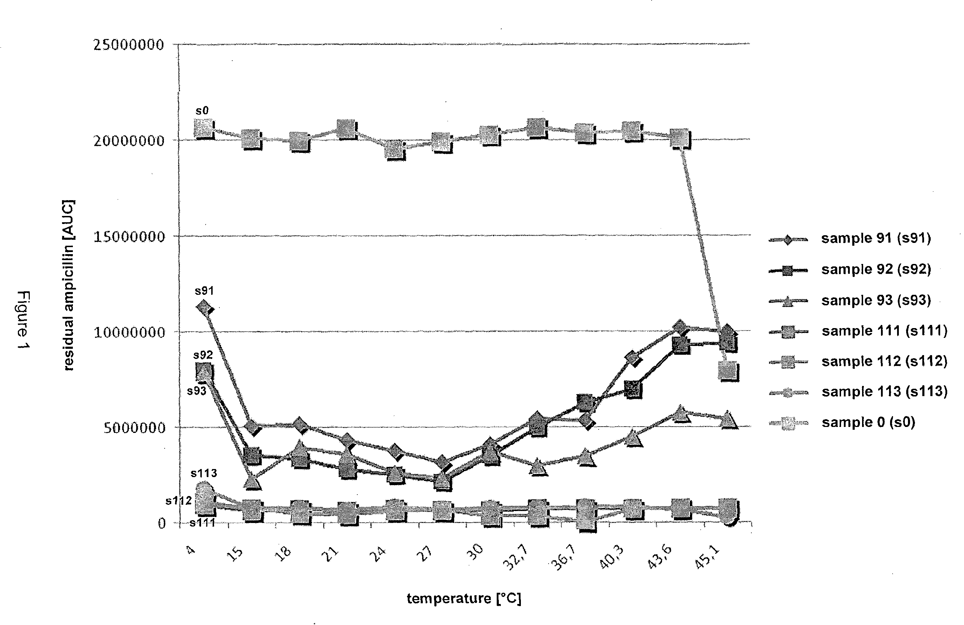

Six subcultured *E. coli* strains (samples 91, 92, 93, 111, 112, and 113) isolated from blood of patients suffering from severe sepsis were tested according to the method of the present invention using ten different incubation temperatures between 15°C and 43°C. Residual ampicillin concentration as area under the curve (AUC) was determined in the supernatant by MAAST. In threes strains (samples 111, 112, and 113), no temperature-dependent changes in residual ampicillin could be found, while in three strains (samples 91, 92, and 93) the residual ampicillin increased between 27°C and 43°C by about 100% (Fig. 1). These *E. coli* strains were in the following characterized as multi-resistant and ESBL-positive, whereas the other three strains (samples 111, 112, and 113) were not multi-resistant. The cutoff for the differentiation is the variation coefficient of MAAST which is about 15%.

## Claims

1. A method for the detection of multiresistant gram-negative bacteria, comprising the steps of:
(a) providing a sample containing gram-negative bacteria;
(b) determining the penicillin- or aminopenicillin-specific activity of beta-lactamases in said bacteria at at least two different temperatures; and
(c) determining whether the bacteria are multiresistant;
wherein the bacteria are multiresistant when the penicillin- or aminopenicillin-specific activity of beta-lactamases determined in step (b) is inversely proportional to the temperature at which said activity has been determined.

2. The method of claim 1, wherein the bacteria contained in the sample are known to express at least one beta-lactamase.

3. The method of claim 1 or claim 2, wherein the bacteria contained in the sample are known to be resistant to ampicillin.

4. The method according to any one of claims 1 to 3, wherein the penicillin-or aminopenicillin-specific activity of beta-lactamases in the bacteria is determined via the speed of the hydrolysis of ampicillin by said bacteria.

5. The method according to any one of claims 1 to 4, wherein the penicillin-or aminopenicillin-specific activity of beta-lactamases in the bacteria is determined via a mass spectrometry-based assay for antibiotic susceptibility testing (MAAST).

6. The method according to any one of claims 1 to 5, wherein the different temperatures at which the penicillin- or aminopenicillin-specific activity of beta-lactamases is determined in step (b) are chosen within the range of from 15°C to 43°C.
